# DEMANDE DE BREVET EUROPEEN

(11) **EP 2 907 584 A1**
(43) Date de publication de la demande: **19.08.2015**
(21) Numéro de dépôt: 15154395.6
(22) Date de dépôt: 09.02.2015
(51) Int. Cl.: B05B 11/00, B05B 11/04, B65D 81/24, B65D 47/24, B65D 1/32, A45D 34/00, A01N 61/00, C11D 3/42, C11D 3/48, C09K 11/00

(54) **Récipient comprenant un réservoir et un système de distribution de produit**

(30) Priorité: 12.02.2014 FR 1400390
(71) Demandeur: Albéa le Tréport, 76470 Le Tréport (FR)
(72) Inventeur: Clerget, Bernard, 60510 Haudivillers (FR); Elmeguenni, Mohamed, 80350 Mers les Bains (FR)
(74) Mandataire: Sayettat, Julien Christian

(57) **Abrégé**

L'invention concerne un récipient comprenant un réservoir (1) dans lequel un produit est destiné à être conditionné, ledit récipient étant équipé d'un système de distribution dudit produit qui présente un chemin d'amenée (2) dudit produit depuis ledit réservoir jusqu'à un orifice de sortie (3) débouchant dans une zone de distribution (4), ledit récipient présentant au moins une paroi (1, 14, 16) délimitant ledit réservoir, ledit chemin d'amenée et/ou ladite zone de distribution et qui est exposée à la lumière extérieure lors de l'utilisation dudit récipient, ladite paroi étant réalisée à base d'un matériau qui présente des propriétés de photoluminescence après exposition à la lumière extérieure, le rayonnement photoluminescent étant apte à assurer une action microbiocide par irradiation du produit disposé au voisinage de ladite paroi.

## Description

L'invention concerne un récipient comprenant un réservoir dans lequel un produit est destiné à être conditionné, ledit récipient étant équipé d'un système de distribution dudit produit qui présente un chemin d'amenée dudit produit depuis ledit réservoir jusqu'à un orifice de sortie débouchant dans une zone de distribution.

Dans une application particulière, le produit est de type lotion, gel ou crème, par exemple pour une utilisation en cosmétique ou pour des traitements pharmaceutiques.

Le système de distribution peut comprendre une tête dans laquelle l'orifice de sortie est formé au travers d'une zone de distribution, ladite tête étant montée sur ledit récipient en mettant ledit orifice en communication avec le réservoir par l'intermédiaire du chemin d'amenée dans lequel le produit est mis en pression pour être distribué au travers de l'orifice de sortie par exemple sous la forme d'une noisette ou d'un flot continu.

Selon une réalisation, la tête est de type bouton poussoir pour actionner le déplacement du tube d'amenée d'une pompe sur une course de distribution / aspiration du produit sous pression. Selon une autre réalisation, la tête est alimentée en produit sous pression par déformation manuelle réversible du réservoir.

A travers le monde diverses directives visent à règlementer, maîtriser et limiter la présence de substances potentiellement dangereuses pour la santé humaine dans les produits notamment cosmétiques. L'une d'elles est la directive européenne REACh (Registration Evaluation and Authorisation of Chemicals). Aussi, une tendance environnementale pousse les cosméticiens à limiter, voire supprimer de leurs formules les conservateurs qui sont souvent cause d'allergies ou d'intolérances.

Les produits cosmétiques deviennent donc de plus en plus fragiles. En particulier, ils supportent difficilement le stress mécanique ou thermique (causant par exemple un déphasage), le contact de l'air (causant par exemple un dessèchement, une oxydation), et sont facilement contaminables par les bactéries et les champignons.

Pour lutter contre la contamination, les formulateurs tentent de renforcer l'activité conservatrice intrinsèque de leurs produits en ajoutant des ingrédients ayant une activité conservatrice comme certaines huiles essentielles, des essences d'orange, de la vitamine C,... qui ne sont pas déclarés en tant que conservateurs. Aussi, ils limitent l'activité libre de l'eau qu'ils tentent de maintenir basse (AW<0,6) afin que les bactéries ne se développent pas ou peu. Mais les formulateurs butent rapidement sur les limites d'une telle stratégie.

D'un autre côté, tant au niveau du réservoir dans lequel le produit est conditionné qu'au niveau du système de distribution, des récipients protecteurs apparaissent sur le marché. En particulier, les récipients doivent empêcher la contamination microbiologique du produit, non seulement lors du stockage mais surtout entre deux utilisations.

Pour ce faire, on a proposé des systèmes de distribution dont les canaux de sortie sont peu étroits et peu tortueux afin de ne pas soumettre le produit à des pressions lors de sa distribution. On a proposé également des systèmes de distribution sans reprise d'air, dits « airless », dans lesquels l'air n'entre pas en compensation du volume de produit distribué, ou bien dont l'air entrant est filtré à travers une maille de l'ordre de 0,2 µm pour retenir les bactéries et les champignons.

On a proposé également des systèmes de distribution dont l'orifice de sortie est auto-occultant, limitant ainsi le contact du produit avec l'air entre deux distributions. Mais cette auto-occultation mécanique, constituée à l'interface de deux géométries souvent imparfaites, ne peut prétendre à la complète rétention des bactéries et champignons dont la taille est de quelques dixièmes de micromètres.

Pour résoudre ces problèmes de contamination, on a proposé des systèmes de distribution utilisant des matériaux contenant des agents antimicrobiens, par exemple sur base organique tel que le Trichlosan (dénomination commerciale de la Sté Melcoplast) ou sur base argent, lesdits matériaux étant localisés dans une zone de contact avec le produit pour empêcher la contamination du film de produit résiduel contenu dans et autour de l'orifice de sortie.

Mais ces agents antimicrobiens, ajoutés aux matières constitutives du système de distribution, peuvent migrer dans le produit, c'est-à-dire que le produit se charge en quantité non-maîtrisée de conservateurs alors que le formulateur se l'interdisait. En outre, l'activité des agents antimicrobiens organiques se réduit fortement après une radio-réticulation de stérilisation du produit, et aussi par vieillissement naturel.

Le document WO-2013/072635 propose l'utilisation de cuivre métallique qui, de par ses propriétés microbiostatiques, empêche la prolifération voire élimine les contaminants, et ce sans migration dans le produit d'un quelconque agent antimicrobien. Toutefois, cette réalisation nécessite un contact entre le cuivre et le produit à décontaminer, ce qui peut être contraignant pour certaines applications, notamment lorsque les surfaces de contact doivent pouvoir se déformer.

L'invention vise à perfectionner l'art antérieur en proposant notamment un récipient à propriété antimicrobienne fiable dans le temps et dans lequel aucun agent antimicrobien n'est susceptible de migrer dans le produit à distribuer.

En outre, l'invention propose un récipient à propriété antimicrobienne améliorée de sorte à pouvoir distribuer des produits dont la teneur en conservateurs est limitée voire nulle. Par conséquent, l'utilisation combinée d'un récipient selon l'invention avec un tel type de produit est particulièrement avantageuse.

L'utilisation combinée d'un récipient selon l'invention avec un produit à distribuer est également particulièrement avantageuse lorsque l'activité conservatrice intrinsèque dudit produit est améliorée, par exemple en ajoutant des ingrédients ayant une activité conservatrice comme certaines huiles essentielles, des essences d'orange, de la vitamine C et/ou en limitant l'activité libre de l'eau (AW<0,6).

Pour atteindre ces différents perfectionnements, l'invention propose un récipient comprenant un réservoir dans lequel un produit est destiné à être conditionné, ledit récipient étant équipé d'un système de distribution dudit produit qui présente un chemin d'amenée dudit produit depuis ledit réservoir jusqu'à un orifice de sortie débouchant dans une zone de distribution, ledit récipient présentant au moins une paroi délimitant ledit réservoir, ledit chemin d'amenée et/ou ladite zone de distribution et qui est exposée à la lumière extérieure lors de l'utilisation dudit récipient, ladite paroi étant réalisée à base d'un matériau qui présente des propriétés de photoluminescence après exposition à la lumière extérieure, le rayonnement photoluminescent étant apte à assurer une action microbiocide par irradiation du produit disposé au voisinage de ladite paroi.

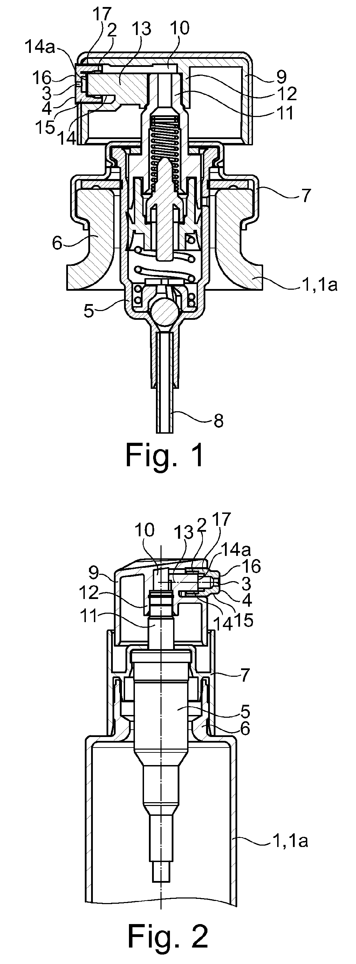

D'autres objets et avantages de l'invention apparaîtront dans la description qui suit, faite en référence aux figures 1 à 4 annexées qui sont des vues partielles en coupe longitudinale d'un récipient selon respectivement un mode de réalisation de l'invention.

En relation avec les figures, on décrit un récipient comprenant un réservoir 1 dans lequel un produit est destiné à être conditionné. Dans un exemple d'application, le produit est une lotion, un gel ou une crème, pour un usage cosmétique ou pour des traitements pharmaceutiques.

Le récipient est équipé d'un système de distribution du produit conditionné dans le réservoir 1, ledit système présentant un chemin d'amenée 2 dudit produit depuis ledit réservoir jusqu'à un orifice de sortie 3 débouchant dans une zone de distribution 4.

En particulier, le système de distribution peut comprendre un organe 5 de prélèvement sous pression du produit contenu dans le réservoir 1, notamment une pompe.

Dans les modes de réalisation représentés, le réservoir 1 comprend un corps surmonté par un col 6 et le système de distribution comprend une frette 7 sur laquelle la pompe 5 est montée, ladite frette étant associée au col 6 de sorte à mettre la pompe 5 en communication étanche avec le produit conditionné dans ledit corps.

En relation avec la figure 1, la pompe 5 comprend un corps dans lequel sont disposés des moyens nécessaires pour la mise sous pression du produit à distribuer. Selon une réalisation particulière, la pompe 5 est de type sans reprise d'air en compensation du volume de produit distribué, de sorte à ne pas introduire de contaminant dans le produit conditionné.

L'invention n'est pas limitée à une structure de pompe 5 particulière, ni à un mode particulier de montage de la pompe 5 sur le réservoir 1. En outre, la pompe 5 peut être alimentée en produit conditionné par l'intermédiaire d'un tube plongeur 8 disposé dans le réservoir 1 ou par l'intermédiaire d'un piston d'amenée du produit qui est monté coulissant dans le réservoir 1 de sorte à pousser le produit dans la pompe 5.

Pour permettre la distribution du produit sous pression, par exemple sous la forme d'une noisette ou d'un flot continu, le système de distribution comprend une tête 9 qui est montée sur le récipient et qui présente une paroi extérieure dans laquelle l'orifice de sortie 3 débouche au travers d'une zone de distribution 4 formée sur ladite paroi, ainsi qu'un canal interne 10 dans lequel s'étend une partie aval du chemin d'amenée 2 entre ledit récipient et ledit orifice de sortie.

En particulier, la pompe 5 comprend un tube d'amenée 11 du produit sous pression, ledit tube étant déplaçable réversiblement sur une course de distribution / aspiration du produit. Pour actionner ce déplacement, la tête 9 de distribution présente un puits 12 de montage de ladite tête sur la partie aval du tube d'amenée 11, une partie du chemin d'amenée 2 s'étendant dans ledit tube entre le récipient et le canal interne 10.

La tête 9 de distribution présente un logement en communication avec le puits 12 de montage par l'intermédiaire du canal interne 10, ledit logement étant équipé d'une enclume 13 ayant une paroi extérieure 14 autour laquelle est disposé un insert 15 en formant un espace dans lequel s'étend le chemin d'amenée 2. Ainsi, en fixant le puits 12 de montage sur le tube d'amenée 11, la distribution du produit est réalisée par appui sur la tête 9 pour actionner le déplacement dudit tube afin d'acheminer le produit conditionné depuis le tube d'amenée 11 vers l'orifice de sortie 3.

Le système de distribution peut également être utilisé pour d'autres types de distribution. En particulier, le réservoir 1 peut comprendre un corps souple, la mise en pression du produit dans le tube d'amenée 11 s'effectuant alors par rapprochement des parois dudit réservoir.

La zone de distribution 4 est formée sur une paroi avant 16 de l'insert 15 qui est disposée en regard d'une portion avant 14a de la paroi extérieure 14 de l'enclume 13 et dans laquelle l'orifice de sortie 3 est réalisé. L'insert 15 et l'enclume 13 présentent des surfaces qui sont destinées à être en contact avec le produit, notamment les surfaces délimitant entre elles le chemin d'amenée 2 dans lequel du produit est immobilisé entre deux distributions, ainsi que la surface extérieure de la paroi avant 16 sur laquelle des souillures de produit distribué sont formées, notamment par étalement dudit produit sur ladite surface lors de sa récupération par le doigt de l'utilisatrice.

Pour limiter la contamination du produit par les bactéries et les champignons, le récipient présente au moins une paroi délimitant le réservoir 1, le chemin d'amenée 2 et/ou la zone de distribution 4 et qui est exposée à la lumière extérieure lors de l'utilisation dudit récipient, ladite paroi étant réalisée à base d'un matériau qui présente des propriétés de photoluminescence après exposition à la lumière extérieure, le rayonnement photoluminescent étant apte à assurer une action microbiocide par irradiation du produit disposé au voisinage de ladite paroi.

Ainsi, lorsque le récipient est utilisé, il est exposé à la lumière du jour pendant quelques instants, ce qui suffit pour charger le matériau de l'énergie qui lui permettra d'être phosphorescent après ladite exposition, par exemple après la remise en place d'un capuchon, l'extinction de la lumière dans la pièce ou la tombée de la nuit.

Par ailleurs, l'utilisation d'un tel matériau permet d'effectuer la décontamination par irradiation d'un volume de produit, notamment sans nécessiter un contact entre le produit à décontaminer et ledit matériau et en permettant d'améliorer l'efficacité de ladite décontamination par action à l'intérieur dudit volume et non seulement par sa surface.

En particulier, le matériau dans lequel la paroi photoluminescente est réalisée peut être choisi de manière à émettre un rayonnement photoluminescent qui présente une longueur d'onde comprise entre 250 et 260 nanomètres, et notamment de 254 nanomètres, ce qui correspond à l'ordre de grandeur des rayonnements ultraviolets stérilisants. Ainsi, la décontamination du volume de produit recevant ce rayonnement photoluminescent peut être réalisée en quelques secondes.

En outre, la paroi photoluminescente peut être réalisée à base d'un matériau comprenant au moins une polyoléfine, par exemple du polyéthylène, du polypropylène et/ou du polystyrène, ledit matériau étant chargé avec au moins un additif présentant des propriétés de photoluminescence, notamment avant la formation de ladite paroi, par exemple par extrusion ou injection.

Par ailleurs, la paroi photoluminescente est translucide à la lumière extérieure, ce qui permet une absorption de la lumière au coeur de ladite paroi et donc une puissance d'irradiation du produit à décontaminer qui est améliorée.

Selon une réalisation, au moins une partie du réservoir 1 est formée à l'intérieur d'une paroi photoluminescente 1a. En particulier, les récipients de petite taille, tels que les échantillons agencés pour une durée d'utilisation courte, peuvent comprendre un réservoir 1 entièrement réalisé en matériau photoluminescent.

En outre, au moins une partie du chemin d'amenée 2 du produit et/ou au moins une partie de la zone de distribution 4 entourant l'orifice de sortie 3 peuvent être réalisées à l'intérieur ou sur une paroi photoluminescente. Ainsi, on assure la décontamination du produit qui, entre deux distributions, est localisé au voisinage de l'orifice de sortie 3 et on empêche la pénétration des bactéries et des champignons dans le réservoir 1 par l'intermédiaire du chemin d'amenée 2.

En variante, du matériau phosphorescent peut entourer le chemin d'amenée 2, par exemple par disposition de pièces rapportées réalisées dans ledit matériau autour des parois délimitant ledit chemin d'amenée, de sorte que le produit disposé dans ledit chemin n'entre pas en contact avec ledit matériau mais soit irradié au travers desdites parois. En particulier, les parois délimitant le chemin d'amenée 2 peuvent alors être réalisées en un matériau transparent quelconque.

Selon une réalisation, la paroi extérieure 14 de l'enclume 13 et/ou la paroi avant 16 de l'insert 15, sur laquelle est formée la zone de distribution 4 et dans laquelle l'orifice de sortie 3 débouche, sont photoluminescentes pour empêcher la contamination des souillures disposées sur elles entre deux distributions, assurant ainsi que le produit distribué ultérieurement ne soit pas contaminé par lesdites souillures. De même, la surface périphérique délimitant l'orifice de sortie 3 est photoluminescente puisque le produit contenu dans ledit orifice entre deux distributions est également en contact avec l'air.

La dimension maximale de l'orifice de sortie 3 peut être inférieure ou égale à 0,5 mm afin de limiter la distance maximale séparant le matériau photoluminescent du produit contenu dans ledit orifice. Ainsi, par irradiation d'un film très mince de produit, on s'assure de la décontamination du produit contenu dans l'orifice de sortie 3. En outre, le chemin d'amenée 2, notamment dans sa partie aval débouchant dans l'orifice de sortie 3, peut présenter une épaisseur qui est inférieure ou égale à 0,5 mm afin que le produit soit laminé sur une épaisseur très réduite avant d'alimenter ledit orifice de sortie.

La portion avant 14a de la paroi extérieure 14 délimite avec la paroi avant 16 un volume destiné à contenir du produit, ledit volume définissant l'extrémité aval du chemin d'amenée 2 qui est en communication directe avec l'orifice de sortie 3. Ce volume étant d'une part, proche de l'orifice de sortie 3 et d'autre part, en contact avec le reste du produit conditionné, il peut être délimité par au moins une paroi réalisée en matériau photoluminescent.

En outre, le volume ainsi délimité peut être inférieur à 0,1 mm³ afin de limiter la distance maximale séparant le matériau photoluminescent du produit contenu dans ledit volume. Ainsi, par irradiation d'un volume très faible de produit, on s'assure de la décontamination du produit contenu dans ledit volume.

Selon une réalisation, l'insert 15 et/ou l'enclume 13 sont réalisés en matériau synthétique, par exemple de type polyoléfine, dont au moins une surface destinée à être en contact avec le produit est chargée avec au moins un additif présentant des propriétés de photoluminescence. En particulier, le taux de charge est suffisant pour pouvoir décontaminer par irradiation le volume de produit stocké entre deux utilisations à l'interface entre l'insert 15 et l'enclume 13.

Dans cette réalisation, lorsqu'une partie de l'insert 15 est visible par l'utilisatrice, on peut prévoir que le matériau photoluminescent soit visible sur ladite partie afin que ladite utilisatrice puisse identifier les propriétés microbiocides du système de distribution.

En relation avec les figures 1, 2 et 4, l'insert 15 est rigide et présente une jupe 17 qui est emmanchée dans le logement, ladite jupe étant refermée vers l'avant par une paroi avant 16 dans laquelle l'orifice de sortie 3 est formé. En outre, l'insert 15 est monté autour de l'enclume 13 pour former entre eux une partie annulaire du chemin d'amenée 2, l'enclume 13 présentant une paroi extérieure 14 dont une portion avant 14a est disposée en regard axial de l'orifice de sortie 3 en formant un volume en communication d'une part, avec ladite partie annulaire et d'autre part, avec l'orifice de sortie 3.

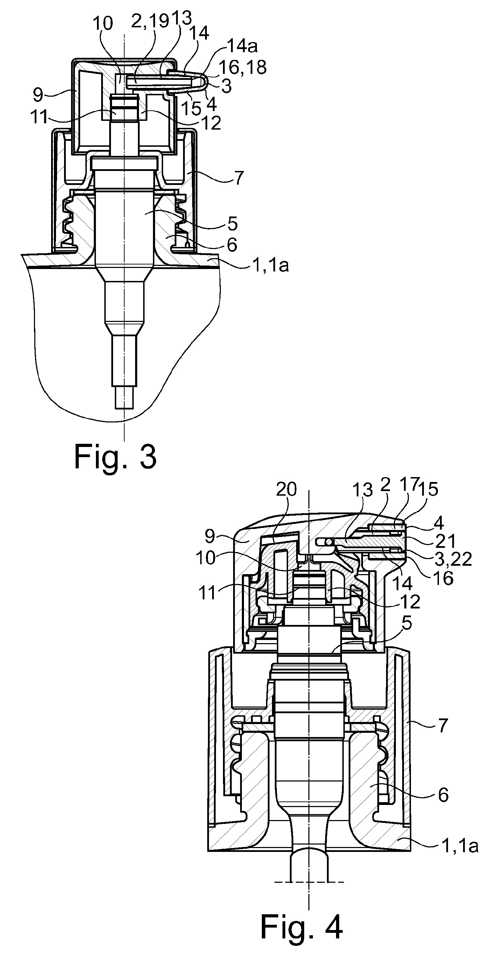

En relation avec la figure 3, l'insert 15 est déformable réversiblement entre un état fermé (figure 3) et un état ouvert de l'orifice de sortie 3. En particulier, l'insert 15 est réalisé en matériau élastomère dans une portée 16 duquel est formée au moins une lèvre 18 déplaçable par application de la pression du produit distribué sur elle, ledit insert étant disposé autour de l'enclume 13 qui peut être rapportée dans la tête 9 (figure 3) ou intégrée à elle. Par ailleurs, l'enclume 13 présente un conduit interne 19 formant le chemin d'amenée 2 qui débouche sur une portion avant 14a disposée en regard de l'orifice de sortie 3.

Dans cette réalisation, au moins la lèvre 18 peut être chargée d'au moins un additif photoluminescent tout en conservant des propriétés d'élasticité suffisantes pour l'insert 15, afin d'assurer la décontamination du film de produit retenu dans ladite lèvre lorsque ledit insert est en état fermé. En variante, on peut prévoir que la lèvre 18 en état fermé soit en contact sur une portée de l'enclume 13 qui comprend un matériau photoluminescent.

Sur la figure 4, l'enclume 13 est déplaçable réversiblement entre une position d'obturation (figure 4) et une position d'ouverture de l'orifice de sortie 3 pour permettre la fermeture réversible dudit orifice entre deux utilisations. Cette figure représente une réalisation connue du document FR-2 948 343 dans laquelle le déplacement de l'enclume 13 est réalisé au moyen d'une pièce 20 présentant le puits 12 de montage, la tête 9 étant montée en coulissement autour de ladite pièce entre une position haute et une position basse, ladite enclume étant montée entre la tête 9 et la pièce 20 par l'intermédiaire d'un dispositif de déplacement réversible de ladite enclume.

Dans cette réalisation, l'enclume 13 présente une tête 21 formant une paroi avant 14 qui, en position d'obturation, est reçue de façon étanche dans un siège 22 formé sur la surface périphérique de l'orifice 3, ladite tête étant disposée à distance dudit siège en position d'ouverture. De façon avantageuse, au moins la paroi 14 de la tête 21 ou le siège 22 comprennent un matériau photoluminescent pour assurer la décontamination du film de produit retenu à l'interface entre eux lorsque l'enclume 13 est en position d'obturation.

## Revendications

1. Récipient comprenant un réservoir (1) dans lequel un produit est destiné à être conditionné, ledit récipient étant équipé d'un système de distribution dudit produit qui présente un chemin d'amenée (2) dudit produit depuis ledit réservoir jusqu'à un orifice de sortie (3) débouchant dans une zone de distribution (4), ledit récipient présentant au moins une paroi (1a, 14, 16) délimitant ledit réservoir, ledit chemin d'amenée et/ou ladite zone de distribution et qui est exposée à la lumière extérieure lors de l'utilisation dudit récipient, ledit récipient étant **caractérisé en ce que** ladite paroi est réalisée à base d'un matériau qui présente des propriétés de photoluminescence après exposition à la lumière extérieure, le rayonnement photoluminescent étant apte à assurer une action microbiocide par irradiation du produit disposé au voisinage de ladite paroi.

2. Récipient selon la revendication 1, **caractérisé en ce que** le rayonnement photoluminescent présente une longueur d'onde comprise entre 250 et 260 nanomètres.

3. Récipient selon l'une des revendications 1 ou 2, **caractérisé en ce que** la paroi photoluminescente (1a, 14, 16) est translucide à la lumière extérieure.

4. Récipient selon l'une quelconque des revendications 1 à 3, **caractérisé en ce que** la paroi photoluminescente (1a, 14, 16) est réalisée à base d'un matériau comprenant au moins une polyoléfine qui est chargée avec au moins un additif présentant des propriétés de photoluminescence.

5. Récipient selon l'une quelconque des revendications 1 à 4, caractérisé en qu'au moins une partie du réservoir (1) est formée à l'intérieur d'une paroi photoluminescente (1a).

6. Récipient selon l'une quelconque des revendications 1 à 5, **caractérisé en ce qu'**au moins une partie du chemin d'amenée (2) du produit est réalisée à l'intérieur d'une paroi photoluminescente (14, 16).

7. Récipient selon l'une quelconque des revendications 1 à 6, **caractérisé en ce qu'**au moins une partie de la zone de distribution (4) entourant l'orifice de sortie (3) est réalisée sur une paroi photoluminescente (16).

8. Récipient selon la revendication 7, **caractérisé en ce que** le système de distribution comprend une tête (9) présentant une paroi extérieure (16) photoluminescente dans laquelle l'orifice de sortie (3) débouche au travers d'une zone de distribution (4) formée sur ladite paroi, ladite tête étant montée sur le récipient en présentant un canal interne (10) dans lequel s'étend une partie aval du chemin d'amenée (2) entre ledit récipient et ledit orifice de sortie.

9. Récipient selon la revendication 8, **caractérisé en ce que** la tête (9) présente un puits (12) de montage de ladite tête sur un tube d'amenée (11) du produit dans lequel s'étend une partie du chemin d'amenée (2) entre ledit récipient et le canal interne (10).

10. Récipient selon l'une des revendications 8 ou 9, **caractérisé en ce que** le système de distribution comprend un organe (5) de prélèvement du produit sous pression au travers du tube d'amenée (11).

11. Récipient selon l'une quelconque des revendications 8 à 10, **caractérisé en ce que** la tête (9) présente un logement équipé d'une enclume (13) ayant une paroi extérieure (14) autour de laquelle est disposé un insert (15) en formant un espace dans lequel s'étend le chemin d'amenée (2), la zone de distribution (4) étant formée sur une paroi avant (16) dudit insert dans lequel est réalisé l'orifice de sortie (3), au moins la paroi extérieure (14) de l'enclume (13) et/ou la paroi avant (16) de l'insert (15) étant photoluminescente.

12. Récipient selon la revendication 11, **caractérisé en ce que** l'insert (15) est déformable réversiblement entre un état fermé et un état ouvert de l'orifice de sortie (3).

13. Récipient selon la revendication 11 ou 12, **caractérisé en ce que** l'enclume (13) est déplaçable réversiblement entre une position d'obturation et une position d'ouverture de l'orifice de sortie (3).

14. Récipient selon l'une quelconque des revendications 1 à 13, **caractérisé en ce qu'**au moins une paroi photoluminescente (14, 16) délimite un volume destiné à contenir du produit, ledit volume étant inférieur à 0,1 mm³.
